# EUROPÄISCHE PATENTSCHRIFT

(11) **EP 1 734 047 B1**
(45) Veröffentlichungstag und Bekanntmachung des Hinweises auf die Patenterteilung: **07.05.2014**
(21) Anmeldenummer: 06010463.5
(22) Anmeldetag: 20.05.2006
(51) Int. Cl.: C07K 1/14, C07K 1/30

(54) **Verwendung von Ionischen Flüssigkeiten zur Proteinextraktion**
Use of ionic liquids for protein extraction
Utilisation des liquides ioniques pour l'extraction de protéines

(30) Priorität: 13.06.2005 DE 102005027172
(43) Veröffentlichungstag der Anmeldung: 20.12.2006
(73) Patentinhaber: Merck Patent GmbH, 64293 Darmstadt (DE)
(72) Erfinder: von Hagen, Jörg, Dr., 64347 Griesheim (DE); Michelsen, Uwe, Dr., 69469 Weinheim (DE)

(56) Entgegenhaltungen:
- WO-A-2004/024279
- WO-A-2005/103070
- DE-A1- 10 240 098
- JAIN N ET AL: "Chemical and biochemical transformations in ionic liquids" TETRAHEDRON, ELSEVIER SCIENCE PUBLISHERS, AMSTERDAM, NL, Bd. 61, Nr. 5, 31. Januar 2005 (2005-01-31), Seiten 1015-1060, XP004714479 ISSN: 0040-4020
- YANG Z ET AL: "Ionic liquids: Green solvents for nonaqueous biocatalysis" ENZYME AND MICROBIAL TECHNOLOGY, STONEHAM, MA, US, Bd. 37, Nr. 1, 1. Juni 2005 (2005-06-01), Seiten 19-28, XP004873852 ISSN: 0141-0229

## Beschreibung

Die vorliegende Erfindung betrifft ein Verfahren zur Extraktion von Proteinen, Proteinfragmenten und/oder Peptiden aus biologischen Proben, wobei als Extraktionsmittel lonische Flüssigkeiten der allgemeinen Formel K⁺A⁻ eingesetzt werden.

Der Nachweis beziehungsweise die Analyse von Proteinen gewinnt in der Medizin zunehmende Bedeutung. Dies betrifft insbesondere die Analyse von Gewebeproben, um Anzeichen einer Erkrankung oder den Nachweis einer krankhaften Veränderung festzustellen. Zu diesem Zwecke werden Analysen des Gesamtproteoms der Zelle untersucht. Hierzu ist es essentiell, alle Proteine einer gegebenen Probe quantitativ zu extrahieren, da nur die Analyse aller beteiligten Proteine in ihrer Gesamtheit Rückschlüsse auf Krankheiten und deren Entstehung zulässt.

Es bestand daher die Aufgabe, Verfahren bereit zu stellen, mit deren Hilfe Proteine aus biologischen Proben extrahiert werden können, um sie weiter analysieren zu können.

Gegenstand der vorliegenden Erfindung sind demgemäss Verfahren zur Extraktion von Proteinen, Proteinfragmenten und/oder Peptiden aus biologischen Proben, wobei als Extraktionsmittel lonische Flüssigkeiten der allgemeinen Formel K⁺A⁻ eingesetzt werden.

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf.

Das Gebiet der ionischen Flüssigkeiten wird zur Zeit intensiv erforscht, da die Anwendungsmöglichkeiten vielfältig sind. Übersichtsartikel zu ionischen Flüssigkeiten sind beispielsweise R. Sheldon "Catalytic reactions in ionic liquids", Chem. Commun., 2001, 2399-2407; M.J. Earle, K.R. Seddon "lonic liquids. Green solvent for the future", Pure Appl. Chem., 72 (2000), 1391-1398; P. Wasserscheid, W. Keim "Ionische Flüssigkeiten - neue Lösungen für die Übergangsmetallkatalyse", Angew. Chem., 112 (2000), 3926-3945; T. Welton "Room temperature ionic liquids. Solvents for synthesis and catalysis", Chem. Rev., 92 (1999), 2071-2083 oder R. Hagiwara, Ya. Ito "Room temperature ionic liquids of alkylimidazolium cations and fluoroanions", J. Fluorine Chem., 105 (2000), 221-227).

WO 2004 024279 offenbart eine Verwendung von Ionischen Flüssigkeiten zur Solubilisierung von insbesondere hydrophoben Proteinen.

Es hat sich überraschend herausgestellt, dass ionische Flüssigkeiten der allgemeinen Formel K⁺A⁻ geeignet sind, Proteine, Proteinfragmente und/oder Peptide aus biologischen Proben zu extrahieren. Dies gilt auch dann, wenn die biologischen Proben fixiert sind, wie es üblicherweise bei der Probenpräparation von biologischen Proben der Fall ist. Das erfindungsgemäße Extraktionsverfahren kann überraschenderweise auch auf fixierte Proben angewendet werden, was die Einsatzmöglichkeiten der Verfahren enorm erweitert. Auf diese Weise lassen sich mit den erfindungsgemäßen Verfahren biologische Proben universell behandeln, um daraus Proteine, Proteinfragmente und/oder Peptide zu extrahieren. Dabei hat das erfindungsgemäße Verfahren den Vorteil, dass die zu extrahierenden Bestandteile nicht degradiert werden, das heißt ihre chemische Konstitution bleibt erhalten. Dies ist ein wesentlicher Vorteil gegenüber bekannten Verfahren, wie sie beispielsweise in W02004/080579 beschrieben sind, bei denen die zu extrahierenden Proteine in einem proteolytischen Schritt zu Peptiden degradiert werden müssen, um sie nachfolgend analysieren zu können. Insgesamt wird damit mit dem erfindungsgemäßen Verfahren eine einfache Aufbereitung biologischer Proben zur Isolierung von Proteinen, Proteinfragmenten und/oder Peptiden möglich, die mit dem aus dem Stand der Technik beschriebenen Verfahren nicht zugänglich war.

Die Extraktion von Proteinen, Proteinfragmenten und/oder Peptiden in den erfindungsgemäßen Verfahren kann aus allen dem Fachmann bekannten biologischen Proben erfolgen. Vorzugsweise handelt es sich bei den biologischen Proben um Gewebe, wie z.B. Biopsien und histologische Präparate, Zellen, Zellkulturen und/oder Körperflüssigkeiten, wie z.B. Blut, Plasma, Serum, Urin, Liquor oder Speichel. Die Extraktion von Proteinen aus Geweben und Zellkulturen erlaubt insbesondere den Nachweis spezifischer Proteine, z.B. zum Nachweis von Proteinen, die auf das Vorhandensein von Krankheiten schließen lassen. Daher ist das erfindungsgemäße Verfahren insbesondere auch für pathologisch interessante Gewebeproben vorteilhaft.

Die biologischen Proben können unfixiert oder fixiert sein, vorzugsweise sind sie fixiert. Dabei kann die Fixierung nach allen dem Fachmann bekannten Methoden erfolgt sein, z.B. durch Einsatz von Formalin, Glutaraldehyd, Formalinsubstituten, Alkoholen, wie z.B. Ethanol, Methanol, Isopropanol, oder inerten Materialien, wie z.B. Paraffin, Harzen oder Polymeren. Vorzugsweise sind die biologischen Proben mit Formalin fixiert. Formalin ist das gängigste Fixierungsmittel bei der Präparation histologischer Proben und wird, wegen des geringen Preises und der guten Fixierungswirkung, vielerorts und nunmehr seit mehr als hundert Jahren eingesetzt. Die Fixierung mit Formalin hat aber den Nachteil, dass es zu einer Quervernetzung der Proteine mit der Probenmatrix kommt. Dadurch wurden die Proteine generell unlöslich und standen einer weiteren Analytik nicht zur Verfügung. Durch Einsatz von lonischen Flüssigkeiten in den erfindungsgemäßen Verfahren können die Proteine jedoch unter Erhalt ihrer Struktur auch aus diesen Proben extrahiert werden, um sie weiter zu analysieren. Auf diese Weise wird die gängigste Fixierungsmethode mit Formalin in ihrer Praktikabilität erweitert, ohne die Möglichkeiten der nachfolgenden Analytik einzuschränken. Damit können nun unter Einsatz der erfindungsgemäßen Verfahren auch Proben mit der modernen Proteinanalytik untersucht werden, die bereits sehr lange mittels Einsatz von Formalin konserviert wurden, das heißt eine zeitliche Begrenzung der Analysierbarkeit von Proben entfällt.

Generell eignen sich in den erfindungsgemäßen Verfahren alle dem Fachmann bekannten lonischen Flüssigkeiten der allgemeinen Formel K⁺A⁻, beispielsweise mit Hexafluorophosphat-, Tetrafluroborat- oder Halogenid-Anionen. Beispiele für geeignete lonische Flüssigkeiten sind Trihexyl(tetradecyl)-phosphoniumhexafluorophosphat, Trihexyl(tetradecyl)-phosphoniumchlorid oder Trihexyl(tetradecyl)-phosphoniumtetrafluoroborat.

Vorzugsweise ist das Anion A- der lonischen Flüssigkeit ausgewählt aus der Gruppe der Imide, insbesondere jene der allgemeinen Formel

[N(R_{f})₂]⁻

wobei R_{f} teilweise oder vollständig mit Fluor substituiertes Alkyl mit 1 bis 8 C-Atomen oder R_{f2}X bedeutet, wobei R_{f2} teilweise oder vollständig mit Fluor substituiertes Alkyl mit 1 bis 8 C-Atomen und X SO₂ oder CO bedeutet, aus der Gruppe der Fluoralkylphosphate, insbesondere jene der allgemeinen Formel

[PFₓ(C_{y}F_{2y+1-z}H_{z})₆₋ₓ]⁻

mit 1 ≤ x < 6, 1 ≤ y ≤ 8 und 0 ≤ z ≤ 2y+1 oder aus Mischungen hieraus.

Vorzugsweise ist R_{f} Trifluormethyl, Pentafluorethyl, Nonaflurobutyl oder R_{f2}SO₂, insbesondere Trifluormethyl oder R_{f2}SO₂, wobei besonders bevorzugt R_{f2} Trifluormethyl ist. Im Falle der Fluoralkylphosphate ist bevorzugt x = 3, z = 0 und y = 2, 3 oder 4, wobei y = 2 ganz besonders bevorzugt ist. lonische Flüssigkeiten enthaltend die besonders bevorzugten [(CF₃SO₂)₂N]⁻- oder [PF₃(C₂F₅)₃]⁻-Anionen sind besonders zur Extraktion von Proteinen, Proteinfragmenten und/oder Peptiden in den erfindungsgemäßen Verfahren geeignet.

In bezug auf die Wahl des Kations K⁺ der lonischen Flüssigkeit der ionischen Flüssigkeit gibt es per se keine Einschränkungen. Vorzugsweise handelt es sich aber um organische Kationen, wobei es sich insbesondere bevorzugt um Ammonium-, Phosphonium, Uronium-, Thiouronium-, Guanidiniumkationen oder um heterocyclische Kationen handelt.

Ammoniumkationen können beispielsweise durch die Formel (1)

[NR₄]⁺ (1),

beschrieben werden, wobei
R jeweils unabhängig voneinander
H, wobei nicht alle Substituenten R gleichzeitig H sein dürfen,
OR', NR'₂, mit der Maßgabe, dass maximal ein Substituent R in Formel (1) OR', NR'₂ ist,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere R teilweise oder vollständig mit Halogenen, insbesondere -F und/oder -Cl, oder teilweise mit -OH, -OR', - CN, -C(O)OH, -C(O)NR'₂, -SO₂NR'₂, -C(O)X, -SO₂OH, -SO₂X, -NO₂, substituiert sein können, und wobei ein oder zwei nicht benachbarte und nicht α-ständige Kohlenstoffatome des R, durch Atome und/oder Atomgruppierungen ausgewählt aus der Gruppe -O-, -S-, -S(O)-, -SO₂-, - SO₂O-, -C(O)-, -C(O)O-, -N⁺R'₂-, -P(O)R'O-, -C(O)NR'-, -SO₂NR'-, - OP(O)R'O-, -P(O)(NR'₂)NR'-, -PR'₂=N- oder -P(O)R'- ersetzt sein können mit R' = H, nicht, teilweise oder perfluoriertes C₁- bis C₆-Alkyl, C₃- bis C₇-Cycloalkyl, unsubstituiertes oder substituiertes Phenyl und X =Halogen sein kann.
Phosphoniumkationen können beispielsweise durch die Formel (2)

   [PR²₄]⁺ (2),

   beschrieben werden, wobei
R² jeweils unabhängig voneinander
H, OR' oder NR'₂
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere R² teilweise oder vollständig mit Halogenen, insbesondere -F und/oder -Cl, oder teilweise mit -OH, -OR', - CN, -C(O)OH, -C(O)NR'₂, -SO₂NR'₂, -C(O)X, -SO₂OH, -SO₂X, -NO₂, substituiert sein können, und wobei ein oder zwei nicht benachbarte und nicht α-ständige Kohlenstoffatome des R², durch Atome und/oder Atomgruppierungen ausgewählt aus der Gruppe -O-, -S-, -S(O)-, -SO₂-, - SO₂O-, -C(O)-, -C(O)O-, -N⁺R'₂-, -P(O)R'O-, -C(O)NR'-, -SO₂NR'-, - OP(O)R'O-, -P(O)(NR'₂)NR'-, -PR'₂=N- oder -P(O)R'- ersetzt sein können mit R' = H, nicht, teilweise oder perfluoriertes C₁- bis C₆-Alkyl, C₃- bis C₇-Cycloalkyl, unsubstituiertes oder substituiertes Phenyl und X =Halogen.

Ausgeschlossen sind jedoch Kationen der Formeln (1) und (2), in denen alle vier oder drei Substituenten R und R² vollständig mit Halogenen substituiert sind, beispielsweise das Tris(trifluormethyl)methylammoniumkation, das Tetra(trifluormethyl)ammoniumkation oder das Tetra(nonafluorbutyl)ammoniumkation.

Uroniumkationen können beispielsweise durch die Formel (3)

[(R³R⁴N)-C(=OR⁵)(NR⁶R⁷)]⁺ (3),

und Thiouroniumkationen durch die Formel (4),

[(R³R⁴N)-C(=SR⁶)(NR⁶R⁷)]⁺ (4),

beschrieben werden, wobei
R³ bis R⁷ jeweils unabhängig voneinander
Wasserstoff, wobei Wasserstoff für R⁵ ausgeschlossen wird,
geradkettiges oder verzweigtes Alkyl mit 1 bis 20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere der Substituenten R³ bis R⁷ teilweise oder vollständig mit Halogenen, insbesondere -F und/oder -Cl, oder teilweise mit -OH, -OR', -CN, -C(O)OH, -C(O)NR'₂, -SO₂NR'₂, -C(O)X, - SO₂OH, -SO₂X, -NO₂, substituiert sein können, und wobei ein oder zwei nicht benachbarte und nicht α-ständige Kohlenstoffatome von R³ bis R⁷ durch Atome und/oder Atomgruppierungen ausgewählt aus der Gruppe -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- oder -P(O)R'- ersetzt sein können mit R' = H, nicht, teilweise oder perfluoriertes C₁- bis C₆-Alkyl, C₃- bis C₇-Cycloalkyl, unsubstituiertes oder substituiertes Phenyl und X =Halogen.

Guanidiniumkationen können durch die Formel (5)

[C(NR⁸R⁹)(NR¹⁰R¹¹)(NR¹²R¹³)]⁺ (5),

beschrieben werden, wobei
R⁸ bis R¹³ jeweils unabhängig voneinander
Wasserstoff, -CN, NR'₂, -OR'
geradkettiges oder verzweigtes Alkyl mit 1 bis 20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet, wobei ein oder mehrere der Substituenten R⁸ bis R¹³ teilweise oder vollständig mit Halogenen, insbesondere -F und/oder -Cl, oder teilweise mit -OH, -OR', -CN, -C(O)OH, -C(O)NR'₂, -SO₂NR'₂, -C(O)X, - SO₂OH, -SO₂X, -NO₂, substituiert sein können, und wobei ein oder zwei nicht benachbarte und nicht α-ständige Kohlenstoffatome von R⁸ bis R¹³ durch Atome und/oder Atomgruppierungen ausgewählt aus der Gruppe -O-, -S-, -S(O)-, -SO₂-, -SO₂O-, -C(O)-, -C(O)O-, -N⁺R'₂-, -P(O)R'O-, -C(O)NR'-, -SO₂NR'-, -OP(O)R'O-, -P(O)(NR'₂)NR'-, -PR'₂=N- oder -P(O)R'- ersetzt sein können mit R' = H, nicht, teilweise oder perfluoriertes C₁- bis C₆-Alkyl, C₃- bis C₇-Cycloalkyl, unsubstituiertes oder substituiertes Phenyl und X = Halogen.

Darüber hinaus können Kationen der allgemeinen Formel (6)

[HetN]⁺ (6)

eingesetzt werden, wobei
HetN⁺ ein heterocyclisches Kation, ausgewählt aus der Gruppe Imidazolium 1H-Pyrazolium 3H-Pyrazolium 4H-Pyrazolium 1-Pyrazolinium 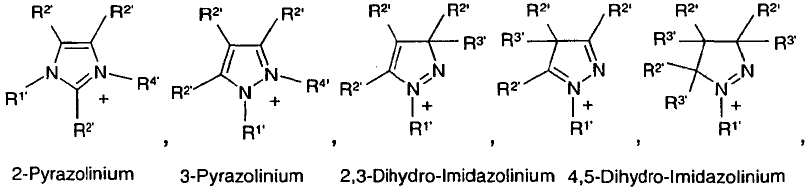 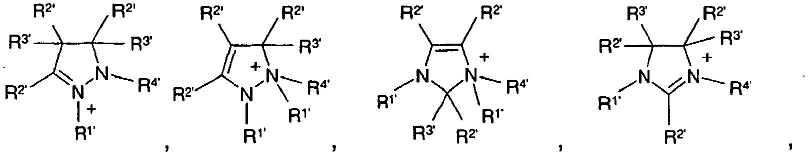 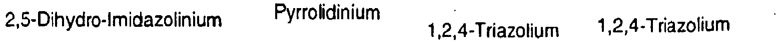 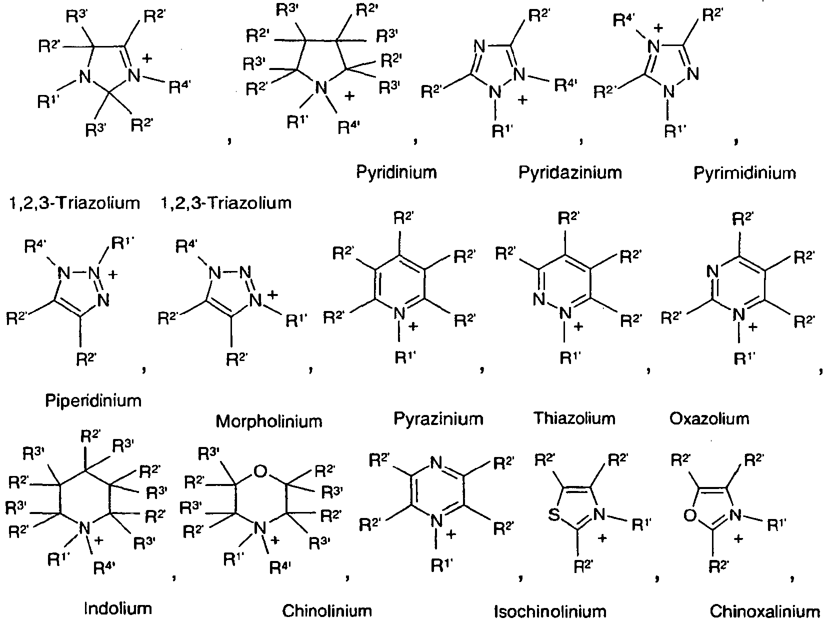 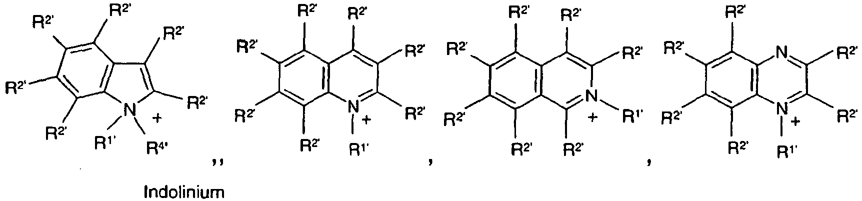 oder 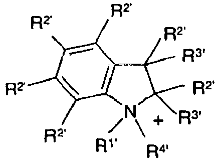 bedeutet, wobei die Substituenten
R^{1'} bis R^{4'} jeweils unabhängig voneinander
Wasserstoff, -CN, -OR', -NR'₂, -P(O)R'₂, -P(O)(OR')₂, -P(O)(NR'₂)₂, - C(O)R', -C(O)OR',
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, gesättigtes, teilweise oder vollständig ungesättigtes Heteroaryl, Heteroaryl-C₁-C₆-alkyl oder Aryl-C₁-C₆-alkyl bedeutet,
wobei die Substituenten R^{1'}, R^{2'}, R^{3'} und/oder R^{4'} zusammen auch ein Ringsystem bilden können,
wobei ein oder mehrere Substituenten R¹' bis R^{4'} teilweise oder vollständig mit Halogenen, insbesondere -F und/oder -Cl, oder -OH, -OR', -CN, -C(O)OH, -C(O)NR'₂, -SO₂NR'₂, -C(O)X, -SO₂OH, -SO₂X, -NO₂, substituiert sein können, wobei jedoch nicht gleichzeitig R^{1'} und R^{4'} vollständig mit Halogenen substituiert sein dürfen, und wobei ein oder zwei nicht benachbarte und nicht am Heteroatom gebundene Kohlenstoffatome der Substituenten R^{1'} bis R^{4'}, durch Atome und/oder Atomgruppierungen ausgewählt aus der -O-, -S-, -S(O)-, -SO₂-, -SO₂O-, -C(O)-, -C(O)O-, - N⁺R'₂-, -P(O)R'O-, -C(O)NR'-, -SO₂NR'-, -OP(O)R'O-, -P(O)(NR'₂)NR'-, - PR'₂=N- oder -P(O)R'- ersetzt sein können mit R' = H, nicht, teilweise oder perfluoriertes C₁- bis C₆-Alkyl, C₃- bis C₇-Cycloalkyl, unsubstituiertes oder substituiertes Phenyl und X =Halogen.

Unter vollständig ungesättigten Substituenten werden im Sinne der vorliegenden Erfindung auch aromatische Substituenten verstanden.

Als Substituenten R und R² bis R¹³ der Verbindungen der Formeln (1) bis (5) kommen erfindungsgemäß dabei neben Wasserstoff bevorzugt in Frage: C₁- bis C₂₀-, insbesondere C₁- bis C₁₄-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl.

Die Substituenten R und R² in den Verbindungen der Formel (1) oder (2) können dabei gleich oder verschieden sein. Bevorzugt sind die Substituenten R und R² verschieden.

Die Substituenten R und R² sind insbesondere bevorzugt Methyl, Ethyl, Isopropyl, Propyl, Butyl, sek.-Butyl, tert.-Butyl, Pentyl, Hexyl, Octyl, Decyl oder Tetradecyl.

Bis zu vier Substituenten des Guanidinium-Kations [C(NR⁸R⁹)(NR¹⁰R¹¹)(NR¹²R¹³)]⁺ können auch paarweise derart verbunden sein, dass mono-, bi- oder polycyclische Kationen entstehen.
Ohne Einschränkung der Allgemeinheit sind Beispiele für solche Guanidinium-Kationen: 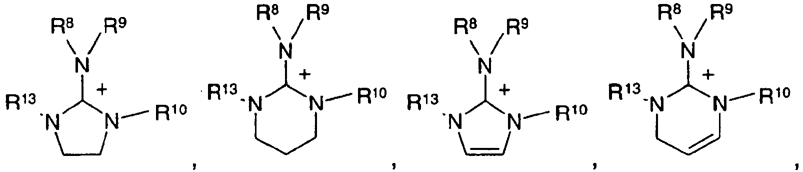 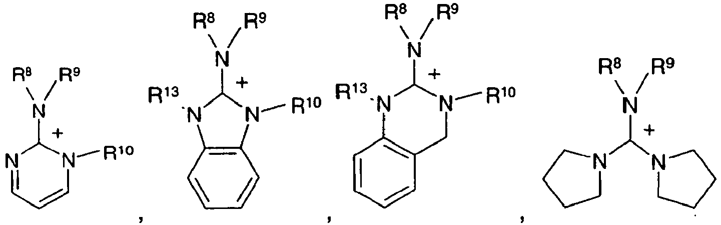 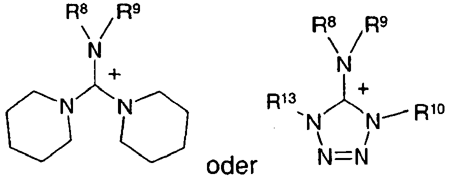 wobei die Substituenten R⁸ bis R¹⁰ und R¹³ eine zuvor angegebene oder besonders bevorzugte Bedeutung haben können.
Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, F, Cl, Br, I, OH, C₁-C₆-Alkoxy, SCF₃, SO₂CF₃, COOH, SO₂NR'₂, SO₂X' oder SO₃H substituiert sein, wobei X und R' eine zuvor angegebene Bedeutung haben, substituiertes oder unsubstituiertes Phenyl oder unsubstituierter oder substituierter Heterocyclus substituiert sein.

Bis zu vier Substituenten des Uroniumkations [(R³R⁴N)-C(=OR⁵)(NR⁶R⁷)]⁺ oder des Thiouroniumkations [(R³R⁴N)-C(=SR⁵)(NR⁶R⁷)]⁺ können auch paarweise derart verbunden sein, dass mono-, bi- oder polycyclische Kationen entstehen.

Beispiele für solche Kationen sind im folgenden angegeben, wobei Y = O oder S bedeutet: 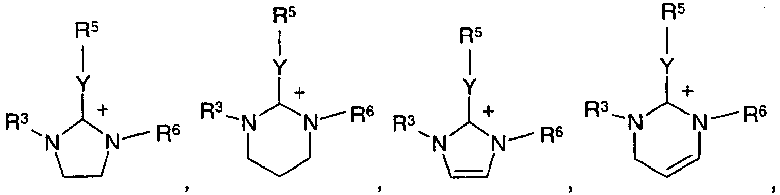 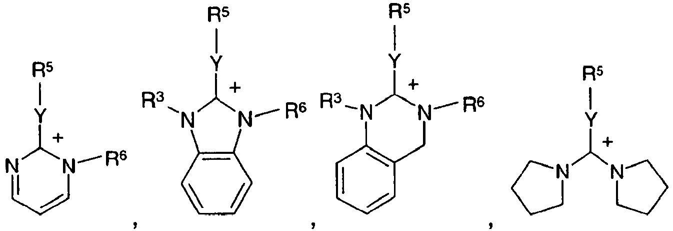 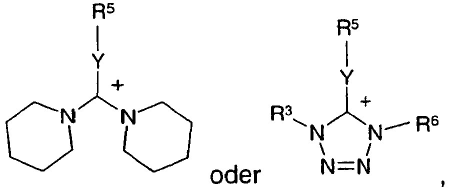 wobei die Substituenten R³, R⁵ und R⁶ eine zuvor angegebene oder besonders bevorzugte Bedeutung haben können.

Gegebenenfalls können die Carbocyclen oder Heterocyclen der zuvor angegebenen Kationen noch durch C₁- bis C₆-Alkyl, C₁- bis C₆-Alkenyl, NO₂, F, Cl, Br, I, OH, C₁-C₆-Alkoxy, SCF₃, SO₂CF₃, COOH, SO₂NR'₂, SO₂X oder SO₃H oder substituiertes oder unsubstituiertes Phenyl oder unsubstituierter oder substituierter Heterocyclus substituiert sein, wobei X und R' eine zuvor angegebene Bedeutung haben.

Die Substituenten R³ bis R¹³ sind jeweils unabhängig voneinander bevorzugt eine geradkettige oder verzweigte Alkylgruppe mit 1 bis 10 C-Atomen. Die Substituenten R³ und R⁴, R⁶ und R⁷, R⁸ und R⁹, R¹⁰ und R¹¹ und R¹² und R¹³ in Verbindungen der Formeln (3) bis (5) können dabei gleich oder verschieden sein. Besonders bevorzugt sind R³ bis R¹³ jeweils unabhängig voneinander Methyl, Ethyl, n-Propyl, Isopropyl, n-Butyl, tert.-Butyl, sek.-Butyl, Phenyl oder Cyclohexyl, ganz besonders bevorzugt Methyl, Ethyl, n-Propyl, Isopropyl oder n-Butyl.

Als Substituenten R^{1'} bis R^{4'} von Verbindungen der Formel (6) kommen erfindungsgemäß dabei neben Wasserstoff bevorzugt in Frage: C₁- bis C₂₀, insbesondere C₁- bis C₁₂-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl.

Die Substituenten R^{1'} und R^{4'} sind jeweils unabhängig voneinander insbesondere bevorzugt Methyl, Ethyl, Isopropyl, Propyl, Butyl, sek.-Butyl, tert.-Butyl, Pentyl, Hexyl, Octyl, Decyl, Cyclohexyl, Phenyl oder Benzyl. Sie sind ganz besonders bevorzugt Methyl, Ethyl, n-Butyl oder Hexyl. In Pyrrolidinium-, Piperidinium- oder Indolinium-Verbindungen sind die beiden Substituenten R^{1'} und R^{4'} bevorzugt unterschiedlich.

Der Substituent R^{2'} oder R^{3'} ist jeweils unabhänigig voneinander insbesondere Wasserstoff, Methyl, Ethyl, Isopropyl, Propyl, Butyl, sek.-Butyl, tert.-Butyl, Cyclohexyl, Phenyl oder Benzyl. Besonders bevorzugt ist R^{2'} Wasserstoff, Methyl, Ethyl, lsopropyl, Propyl, Butyl oder sek.-Butyl. Ganz besonders bevorzugt sind R^{2'} und R^{3'} Wasserstoff.

Die C₁-C₁₂-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl oder Dodecyl. Gegebenenfalls Difluormethyl, Trifluormethyl, Pentafluorethyl, Heptafluorpropyl oder Nonafluorbutyl.

Ein geradkettiges oder verzweigtes Alkenyl mit 2 bis 20 C-Atomen, wobei auch mehrere Doppelbindungen vorhanden sein können, ist beispielsweise Allyl, 2- oder 3-Butenyl, Isobutenyl, sek.-Butenyl, ferner 4-Pentenyl, iso-Pentenyl, Hexenyl, Heptenyl, Octenyl, -C₉H₁₇, -C₁₀H₁₉ bis -C₂₀H₃₉; vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇, vorzugsweise Ethinyl, 1- oder 2-Propinyl, 2- oder 3-Butinyl, 4-Pentinyl, 3-Pentinyl oder Hexinyl.

Aryl-C₁-C₆-alkyl bedeutet beispielsweise Benzyl, Phenylethyl, Phenylpropyl, Phenylbutyl, Phenylpentyl oder Phenylhexyl, wobei sowohl der Phenylring als auch die Alkylenkette, wie zuvor beschrieben teilweise oder vollständig mit Halogenen, insbesondere -F und/oder -Cl, oder teilweise mit -OH, -OR', -CN, -C(O)OH, -C(O)NR'₂, -SO₂NR'₂, -C(O)X, -SO₂OH, -SO₂X, -NO₂ substituiert sein können.

Unsubstituierte gesättigte oder teilweise oder vollständig ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit C₁- bis C₆-Alkylgruppen substituiert sein können, wobei wiederum die Cycloalkylgruppe oder die mit C₁- bis C₆-Alkylgruppen substituierte Cycloalkylgruppe auch mit Halogenatomen wie F, Cl, Br oder I, insbesondere F oder Cl oder mit -OH, -OR', -CN, -C(O)OH, -C(O)NR'₂, -SO₂NR'₂, -C(O)X, -SO₂OH, -SO₂X, -NO₂ substituiert sein kann.

In den Substituenten R, R² bis R¹³ oder R^{1'} bis R^{4'} können auch ein oder zwei nicht benachbarte und nicht α-ständig zum Heteroatom gebundene Kohlenstoffatome, durch Atome und/oder Atomgruppierungen ausgewählt aus der Gruppe -O-, -S-, -S(O)-, -SO₂-, -SO₂O-, -C(O)-, -C(O)O-, -N⁺R'₂-, - P(O)R'O-, -C(O)NR'-, -SO₂NR'-, -OP(O)R'O-, -P(O)(NR'₂)NR'-, -PR'₂=N-oder -P(O)R'- ersetzt werden, mit R' = nicht, teilweise oder perfluoriertes C₁- bis C₆-Alkyl, C₃- bis C₇-Cycloalkyl, unsubstituiertes oder substituiertes Phenyl.

Beispiele für derart modifizierte Substituenten sind R, R² bis R¹³ und R^{1'} bis R^{4'}:
-OCH₃, -OCH(CH₃)₂, -CH₂OCH₃, -CH₂-CH₂-O-CH₃, -C₂H₄OCH(CH₃)₂, - C₂H₄SC₂H₅, -C₂H₄SCH(CH₃)₂, -S(O)CH₃, -SO₂CH₃, -SO₂C₆H₅, -SO₂C₃H₇, - SO₂CH(CH₃)₂, -SO₂CH₂CF₃, -CH₂SO₂CH₃, -O-C₄H₈-O-C₄H₉, -CF₃, -C₂F₅, - C₃F₇, -C₄F₉, -C(CF₃)₃, -CF₂SO₂CF₃, -C₂F₄N(C₂F₅)C₂F₅, -CHF₂, -CH₂CF₃, - C₂F₂H₃, -C₃FH₆, -CH₂C₃F₇, -C(CFH₂)₃, -CH₂C(O)OH, -CH₂C₆H₅, -C(O)C₆H₅ oder P(O)(C₂H₅)₂.

In R' ist C₃- bis C₇-Cycloalkyl beispielweise Cyclopropyl, Cyclobutyl, Cyclopentyl, Cyclohexyl oder Cycloheptyl.

In R' bedeutet substituiertes Phenyl, durch C₁- bis C₆-Alkyl, C₁- bis C₆-Alkenyl, NO₂, F, Cl, Br, I, OH, C₁-C₆-Alkoxy, SCF₃, SO₂CF₃, COOH, SO₂X', SO₂NR"₂ oder SO₃H substituiertes Phenyl, wobei X' F, Cl oder Br und R" ein nicht, teilweise oder perfluoriertes C₁- bis C₆-Alkyl oder C₃- bis C₇-Cycloalkyl wie für R' definiert bedeutet, beispielsweise, o-, m- oder p-Methylphenyl, o-, m- oder p-Ethylphenyl, o-, m- oder p-Propylphenyl, o-, m-oder p-Isopropylphenyl, o-, m- oder p-tert.-Butylphenyl, o-, m- oder p-Nitrophenyl, o-, m- oder p-Hydroxyphenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-Iodphenyl, weiter bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dihydroxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.

In R^{1'} bis R^{4'} wird als Heteroaryl ein gesättigter oder ungesättigter mono-oder bicyclischer heterocyclischer Rest mit 5 bis 13 Ringgliedern verstanden, wobei 1, 2 oder 3 N- und/oder 1 oder 2 S- oder O-Atome vorliegen können und der heterocyclische Rest ein- oder mehrfach durch C₁- bis C₆-Alkyl, C₁- bis C₆-Alkenyl, NO₂, F, Cl, Br, I, OH, C₁-C₆-Alkoxy, SCF₃, SO₂CF₃, COOH, SO₂X', SO₂NR"₂ oder SO₃H substituiert sein kann, wobei X' und R" eine zuvor angegebene Bedeutung haben.
Der heterocyclische Rest ist vorzugsweise substituiertes oder unsubstituiertes 2- oder 3-Furyl, 2- oder 3-Thienyl, 1-, 2- oder 3-Pyrrolyl, 1-, 2-, 4- oder 5-Imidazolyl, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3-oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -4- oder -5-yl, 1- oder 5-Tetrazolyl, 1,2,3-Oxadiazol-4- oder -5-yl 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-1 H-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 1-, 2-, 3-, 4-, 5-, 6-, 7- oder 8-Chinolinyl, 1-, 3-, 4-, 5-, 6-, 7- oder 8-Isochinolinyl, 1-, 2-, 3-, 4-oder 9-Carbazolyl, 1-, 2-, 3-, 4-, 5-, 6-, 7-, 8- oder 9-Acridinyl, 3-, 4-, 5-, 6-, 7- oder 8-Cinnolinyl, 2-, 4-, 5-, 6-, 7- oder 8-Chinazolinyl oder 1-, 2- oder 3-Pyrrolidinyl.

Unter Heteroaryl-C₁-C₆-alkyl wird nun in Analogie zu Aryl-C₁-C₆-alkyl beispielsweise Pyridinyl-methyl, Pyridinyl-ethyl, Pyridinyl-propyl, Pyridinylbutyl, Pyridinyl-pentyl, Pyridinyl-hexyl verstanden, wobei weiterhin die zuvor beschriebenen Heterocyclen in dieser Weise mit der Alkylenkette verknüpft werden können.

### HetN⁺ ist bevorzugt

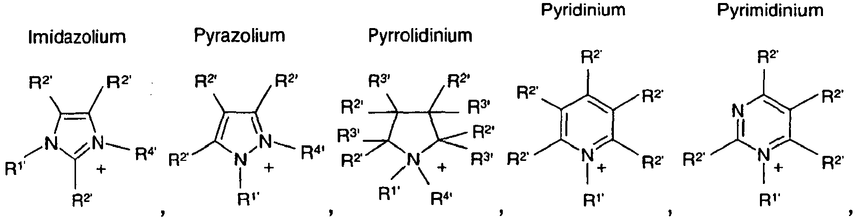

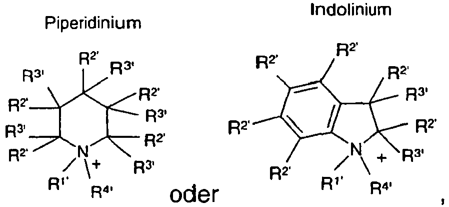

wobei die Substituenten R^{1'} bis R^{4'} jeweils unabhängig voneinander eine zuvor beschriebene Bedeutung haben.

Vorzugsweise handelt es sich bei den Kationen der erfindungsgemäßen ionischen Flüssigkeit um Ammonium-, Phosphonium-, Imidazolium-, Pyridinium- oder Pyrrolidinium-Kationen.

Ganz besonders bevorzugt werden Ammonium-, Phosphonium-, Imidazolium-, Pyridinium- oder Pyrrolidiniumtrifluormethylsulfonylimide, oder Ammonium- oder Phosphoniumtris(pentafluorethyl)trifluorophosphate zur Extraktion in den erfindungsgemäßen Verfahren eingesetzt, wobei Trihexyl(tetradecyl)-phosphonium-hexafluorophosphat, Trihexyl(tetradecyl)-phosphonium-chlorid, Trihexyl(tetradecyl)-phosphonium-tetrafluorborat, N-(3-Hydroxypropyl)-N-methylpyrrolidinium-bis-(trifluormethylsulfonyl)imid, N-(Ethoxymethyl)-N-methylpyrrolidinium-bis-(trifluormethylsulfonyl)imid, N-(2-Methoxyethyl)-N-methylpyrrolidinium-bis-(trifluormethylsulfonyl)imid, N-(2-Methoxyethyl)pyridinium-bis-(trifluormethylsulfonyl)imid, N-Ethyl-4-(N,N-dimethylammonium)pyridinium-bis-(trifluormethylsulfonyl)imid, N-Butyl-4-(N,N-dimethylammonium)pyridinium-bis-(trifluormethylsulfonyl)imid, N-Hexyl-4-(N,N-dimethylammonium)pyridinium-bis-(trifluormethylsulfonyl)imid, 1-(3-Hydroxypropyl)-3-methyl-imidazolium-bis-(trifluormethylsulfonyl)imid, 1-(Ethoxymethyl)-3-methyl-imidazolium-bis-(trifluormethylsulfonyl)imid, 1-(2-Methoxyethyl)-3-methyl-imidazolium-bis-(trifluormethylsulfonyl)imid, 1-(2-Ethoxyethyl)-3-methyl-imidazolium-bis-(trifluormethylsulfonyl)imid, Tetra-butyl-ammonium-bis-(trifluormethylsulfonyl)imid, Tetra-ethyl-ammonium-bis-(trifluormethylsulfonyl)imid, Trihexyl(tetradecyl)ammonium-trifluormethylsulfonylimid, Methyl-trioctyl-ammonium-bis-(trifluormethylsulfonyl)imid, (Ethoxymethyl)ethyldimethylammonium-bis-(trifluormethylsulfonyl)imid, (2-Ethoxymethyl)ethyldimethylammonium-bis-(trifluormethylsulfonyl)imid, (2-Ethoxyethyl)ethyldiethylammonium-bis-(trifluormethylsulfonyl)imid, Ehyldimethylpropylammonium-bis-(trifluormethylsulfonyl)imid, Trihexyl(tetradecyl)-phosphonium-bis(trifluormethylsulfonyl)imid, Trihexyl(tetradecyl)-phosphonium-tris(pentafluorethyl)trifluorphosphat, Tetrabutyl-phosphonium-tris(pentafluorethyl)trifluorphosphat, Tetra-methyl-ammonium-tris(pentafluorethyl)trifluorphosphat, Tetra-ethyl-ammonium-tris(pentafluorethyl)trifluorphosphat oder Tetra-butyl-ammonium-tris(pentafluorethyl)trifluorphosphat, und besonders bevorzugt N-(3-Hydroxypropyl)-N-methylpyrrolidinium-bis-(trifluormethylsulfonyl)imid, N-(Ethoxymethyl)-N-methylpyrrolidinium-bis-(trifluormethylsulfonyl)imid, Trihexyl(tetradecyl)ammoniumtrifluormethylsulfonylimid, Trihexyl(tetradecyl)phosphoniumtrifluormethylsulfonylimid oder Tetra-butyl-ammonium-tris(pentafluorethyl)trifluorphosphat, besonders gute Ergebnisse bei den erfindungsgemäßen Verfahren liefern.

Die erfindungsgemäßen Verfahren können bei Temperaturen von 0 bis 95°C erfolgen, vorzugsweise bei 55 bis 65°C und ganz besonders bevorzugt bei 60°C. Bei den bevorzugten Temperaturen wird eine verbesserte Extraktion in relativ kurzer Zeit beobachtet. Zur schonenden Extraktion, insbesondere von empfindlicheren Proteinen, Proteinfragmenten und/oder Peptiden empfiehlt sich die Durchführung des erfindungsgemäßen Verfahrens bei tieferen Temperaturen innerhalb des angegebenen Temperaturintervalls, unter Berücksichtigung einer dafür erforderlichen längeren Extraktionszeit.

Die erfindungsgemäßen Verfahren eignen sich zur Extraktion von Proteinen, Proteinfragmenten und/oder Peptiden aus biologischen Proben unter Erhalt der zellularen Grundstruktur der Proben. Darüber hinaus ist aber auch die Anwendung der erfindungsgemäßen Verfahren auf entsprechend vorbehandelte Proben denkbar, bei denen z.B. die zellulare Grundstruktur vor Anwendung der erfindungsgemäßen Verfahren zerstört wurde. Diese Vorbehandlung kann auf alle dem Fachmann bekannten Arten erfolgen, z.B. durch manuelle Homogenisierung oder durch Vortexing.

Die mit Hilfe der erfindungsgemäßen Verfahren gewonnenen Extrakte enthalten Proteine, Proteinfragmente und/oder Peptide. Diese eignen sich für alle dem Fachmann bekannten Arten von Proteinanalytik, z.B. Elektrophorese (z.B. 2-dimensionale Gelelektrophorese), immunchemische Nachweismethoden (z.B. Western Blot Analyse, ELISA, RIA), Protein-Arrays (z.B planare und beadbasierende Systeme), Massenspektrometrie (z.B. Maldi, Esi und Seldi) sowie alle biochromatographischen Trennverfahren (IEX, SEC, HIC und Affinitätschromatographie).

### Beispiele

### Beispiel 1

1 g einer mit Formalin fixierten Gewebeprobe (z.B. Lunge, Herz, Niere, Leber, Milz) wird mehrmals mit 10 ml isotonischer Phosphat-gepufferter Kochsalzlösung gewaschen und mit einem Skalpell zerkleinert. 0.1 g der zerkleinerten Probe werden mit 60 µl Trihexyl(tetradecyl)phosphonium-trifluormethylsulfonylimid vermischt und über einen Zeitraum von zwei Stunden in einem Rotationsmischer bei 60°C inkubiert. Die überstehende Lösung wird abgetrennt und kann zur weiteren Analytik der darin enthaltenen Proteine eingesetzt werden.

### Proteinanalytik

Mit dem in Beispiel 1 erhaltenen Extrakt wird eine SDS-Polyacrylamid-Gelelektrophorese wie folgt durchgeführt: 3 µl des Proteinextraktes werden mit 16 µl SDS Probenauftragspuffer (3M Tris pH 8.5, Glyzerin 24 % (v/v), Natriumdodecylsulfat 8% (w/v), 2-Mercaptoethanol 2%, Bromphenolblau 0.1 % (w/v)) vermischt und 5 Minuten bei 95°C inkubiert. Nach Zentrifugation bei 10000xg wird die Probe auf ein 10% SDS Page aufgetragen und 90 Minuten bei 120 Volt aufgetrennt.
Zur Identifizierung der extrahierten Proteine wird ein Westernblot des Gels unter Verwendung von Antikörpern, beispielsweise gegen das Membranprotein Calnexin (90 kDa, Anti-Calnexin, C-terminal, 1:2000, Calbiochem Cat.No: 208880) durchgeführt. In der Westernblot-Analyse wird Calnexin in seiner nativen molekularen Größe identifiziert.

Insgesamt zeigt sich, dass sich die erfindungsgemäßen Verfahren in vorteilhafter Weise zur Extraktion von Proteinen aus biologischen Proben eignen.

## Patentansprüche

1. Verfahren zur Extraktion von Proteinen, Proteinfragmenten und/oder Peptiden aus biologischen Proben, **dadurch gekennzeichnet, dass** als Extraktionsmittel lonische Flüssigkeiten der allgemeinen Formel K⁺A⁻ eingesetzt werden.

2. Verfahren gemäß Anspruch 1, **dadurch gekennzeichnet, dass** es sich bei den biologischen Proben um Gewebe, Zellen, Zellkulturen und/oder Körperflüssigkeiten handelt.

3. Verfahren gemäß Anspruch 1 oder 2, **dadurch gekennzeichnet, dass** die biologischen Proben fixiert sind.

4. Verfahren gemäß Anspruch 3, **dadurch gekennzeichnet, dass** die biologischen Proben mit Formalin fixiert sind.

5. Verfahren gemäß einem der Ansprüche 1 bis 4, **dadurch gekennzeichnet, dass** die Anionen A⁻ der lonischen Flüssigkeit ausgewählt sind aus der Gruppe der Imide der allgemeinen Formel [N(R_{f})₂]⁻ wobei R_{f} teilweise oder vollständig mit Fluor substituiertes Alkyl mit 1 bis 8 C-Atomen oder R_{f2}X bedeutet, wobei R_{f2} teilweise oder vollständig mit Fluor substituiertes Alkyl mit 1 bis 8 C-Atomen und X SO₂ oder CO bedeutet, aus der Gruppe der Fluoralkylphosphate der allgemeinen Formel [PFₓ(C_{y}F_{2y+1-z}H_{z})₆₋ₓ]⁻ mit 1 ≤ x < 6, 1 ≤ y ≤ 8 und 0 ≤ z ≤ 2y+1 oder aus Mischungen hieraus.

6. Verfahren gemäß einem der Ansprüche 1 bis 5, **dadurch gekennzeichnet, dass** die Kationen K⁺ der lonischen Flüssigkeit ausgewählt sind aus der Gruppe der Ammonium-, Phosphonium, Uronium-, Thiouronium-, Guanidiniumkationen oder heterocyclischen Kationen.

7. Verfahren gemäß einem der Ansprüche 1 bis 6, **dadurch gekennzeichnet, dass** die Extraktion bei Temperaturen von 0 bis 95°C erfolgt.

## Claims

1. Method for the extraction of proteins, protein fragments and/or peptides from biological samples, **characterised in that** the extractants employed are ionic liquids of the general formula K⁺A⁻.

2. Method according to Claim 1, **characterised in that** the biological samples are tissues, cells, cell cultures and/or body fluids.

3. Method according to Claim 1 or 2, **characterised in that** the biological samples have been fixed.

4. Method according to Claim 3, **characterised in that** the biological samples have been fixed using formalin.

5. Method according to one of Claims 1 to 4, **characterised in that** the anions A⁻ of the ionic liquid are selected from the group of the imides of the general formula (N(R_{f})₂]⁻, where R_{f} denotes partially or fully fluorine-substituted alkyl having 1 to 8 C atoms or R_{f2}X, where R_{f2} denotes partially or fully fluorine-substituted alkyl having 1 to 8 C atoms and X denotes SO₂ or CO, from the group of the fluoroalkylphosphates of the general formula [PFₓ(C_{y}F_{2y+1-z}H_{z})₆₋ₓ]⁻, where 1 ≤ x < 6, 1 ≤ y ≤ 8 and 0 ≤ z ≤ 2y+1, or from mixtures thereof.

6. Method according to one of Claims 1 to 5, **characterised in that** the cations K⁺ of the ionic liquid are selected from the group of ammonium, phosphonium, uronium, thiouronium, guanidinium and heterocyclic cations.

7. Method according to one of Claims 1 to 6, **characterised in that** the extraction is carried out at temperatures of 0 to 95°C.

## Revendications

1. Méthode d'extraction de protéines, de fragments de protéines et/ou de peptides à partir d'échantillons biologiques, **caractérisée en ce que** les agents d'extraction employés sont des liquides ioniques de formule générale K⁺A⁻.

2. Méthode selon la revendication 1, **caractérisée en ce que** les échantillons biologiques sont des tissus, des cellules, des cultures cellulaires et/ou des fluides corporels.

3. Méthode selon la revendication 1 ou 2, **caractérisée en ce que** les échantillons biologiques ont été fixés.

4. Méthode selon la revendication 3, **caractérisée en ce que** les échantillons biologiques ont été fixés à l'aide de formaldéhyde.

5. Méthode selon l'une des revendications 1 à 4, **caractérisée en ce que** les anions A⁻ du liquide ionique sont choisis dans le groupe constitué par les imides de formule générale [N(R_{f})₂]⁻, où R_{f} désigne alkyle partiellement ou totalement substitué par fluor ayant de 1 à 8 atomes de C ou R_{f2}X, où R_{f2} désigne alkyle partiellement ou totalement substitué par fluor ayant de 1 à 8 atomes de C et X désigne SO₂ ou CO, dans le groupe constitué par les phosphates de fluoroalkyle de formule générale [PFₓ(C_{y}F_{2y+1-z}H_{z})₆₋ₓ]⁻, où 1 ≤ x < 6, 1 ≤ y ≤ 8 et 0 ≤ z ≤ 2y+1, ou des mélanges de ceux-ci.

6. Méthode selon l'une des revendications 1 à 5, **caractérisée en ce que** les cations K⁺ du liquide ionique sont choisis dans le groupe constitué par les cations ammonium, phosphonium, uronium, thiouronium, guanidinium et hétérocycliques.

7. Méthode selon l'une des revendications 1 à 6, **caractérisée en ce que** l'extraction est effectuée à des températures allant de 0 à 95°C.
